Europäisches Patentamt

European Patent Office

Office européen des brevets

(11) Publication number: **0 398 525**
**A1**

## EUROPEAN PATENT APPLICATION

(21) Application number: 90304519.3

(22) Date of filing: 26.04.90

(51) Int. Cl.⁵: **A61K 35/78, A61K 31/66**

(30) Priority: 28.04.89 JP 110323/89

(43) Date of publication of application:
**22.11.90 Bulletin 90/47**

(84) Designated Contracting States:
**AT BE CH DE DK ES FR GB GR IT LI LU NL SE**

(71) Applicant: **SANWA KAGAKU KENKYUSHO CO., LTD.**
**No. 35, Higashi-sotobori-cho**
**Higashi-ku Nagoya-shi Aichi-ken(JP)**

(72) Inventor: **Sawai, Kiichi, c/o Sanwa Kagaku**
**Kenkyusho Co. Ltd.**
**Higashi-Sotobori-cho 35, Higashi-ku**
**Nagoya-shi, Aichi-ken(JP)**
Inventor: **Kurono, Masayasu c/o Sanwa**
**Kagaku Kenkyusho Co.Ltd**
**Higashi-Sotobori-cho 35, Higashi-ku**
**Nagoya-shi, Aichi-ken(JP)**
Inventor: **Asai, Hiromoto, c/o Sanwa Kagaku**
**Kenkyusho Co. Ltd**
**Higashi-Sotobori-cho 35, Higashi-ku**
**Nagoya-shi, Aichi-ken(JP)**
Inventor: **Mitani, Takahiko, c/o Sanwa Kagaku**
**Kenkyusho Co.**
**Higashi-Sotobori-cho 35, Higashi-ku**
**Nagoya-shi, Aichi-ken(JP)**
Inventor: **Ninomiya, Naohisa, c/o Sanwa**
**Kagaku Kenkyusho Co.**
**Higashi-Sotobori-cho 35, Higashi-ku**
**Nagoya-shi, Aichi-ken(JP)**

(74) Representative: **Diamond, Bryan Clive et al**
**Gee & Co., Chancery House, Chancery Lane**
**London WC2A 1QU(GB)**

(54) Compositions, containing inositol phosphate, preparation and uses.

(57) Cereal shells and germs, e. g. of rice bran, are compressed to remove the oily components, dried for as short a time as possible, and extracted with water at pH of 7 or below, e. g. with aqueous hydrochloric acid at a temperature of 80°C or higher, and the solids removed. The extract is dried, optionally after treatment with ethanol.

The product contains inositol phosphate compounds (mono- to hexa-phosphates of inositol) with some saccharides and small amounts of protein, fat and inorganic salts. It, can be formed into pharmaceutical compositions for oral administration, which are useful for removal of body odor or as an antidote to the effect of drugs or alcohol, or it can be used directly as a food enricher.

## COMPOSITIONS CONTAINING INOSITOL PHOSPHATES, THEIR PREPARATION AND USES

The present invention relates to a novel composition containing as a primary ingredient inositol phosphate compounds, to the method of obtaining the compositions from cereal shells and germs, and to pharmaceutical preparations thereof which are useful especially in the removal of body smell and uraroma and as an antidote to drugs and alcohol.

Phytic acid is a hexaphosphate of inositol, and occurs naturally in various kinds of plants in the form of a mixed combined salt with calcium, magnesium and potassium.

Phytic acid and its salts have already been used as pharmaceuticals. For instance, calcium phytate has been used as calcium enhancer; sodium phytate for the prevention of the recurrence of calculus; and potassium phytate for the treatment of hypercalcemia.

In the form of food additives, phytic acid has also been used to prevent the blackening of canned foods and the formation of strabite therein, prevent the discoloration of fruit juice and drinking water, promote fermentation and prevent the oxidation of edible oils as well as for other purposes.

We have found that oral administration of phytic acid is effective for the removal of uraroma and body smell, detoxication, the treatment of diabetes and hyperlipemia, the remediation of erythroclasis, dysmnesia and hypohepatia and the inhibition of the proliferation of fat cells, and that such functions are further enhanced by the addition of ferric ions to phytic acid (see our EP-A-03442955, 0342956, 0344997 and EPA 90303397).

It has been found, on the other hand, that the administration of phytic acid gives rise to a lowering of the absorption of metal ions simultaneous with a metal deficiency, because phytic acid (inositol hexaphosphate) bonds to metal ions such as $Ca^{2+}$, $Zn^{2+}$, $Fe^{2+}$ and $Fe^{3+}$ to form insoluble chelates and salts - see Eiji Miyazawa and Tsutomu Yoshida, "Progress in Medical Science", Vol. 144, No. 10 (1988).

The correlation between inositol phosphate compounds and biometabolism are now being elucidated - see "Progress in Medical Science" Vol. 146, No. 12, page 837, (1988).

We have now made further studies for the purpose of obtaining a useful composition containing as a primary ingredient various inositol phosphate compounds. It has now unexpectedly been found that cereal shells and germs, heretobefore rejected as substantially useless, yield a matter containing large amounts of inositol phosphate compounds, when oily components are squeezed therefrom by compression, and said matter is dried by heating to prevent fermentation just after compression, thereby obtaining inositol phosphate compositions having a pharmacological action, especially producing a strong action of deodorization and detoxication of drugs acting on the central nervous system.

Thus, the present invention relates to a novel composition containing as a primary ingredient inositol phosphate compounds obtained by degreasing cereal shells and germs by compression, drying the compressed matter by heating and extracting the dried matter with water in an acidic range of pH 7 or below.

The invention also relates to the use of the composition, e. g. a pharmaceutical preparation making use of its action for the removal of uraroma and body smell, or detoxication.

For the cereal shells and germs in the present invention, rice bran may typically be used.

The compressed and degreased matter may be obtained by squeezing out of the cereal shells and germs the oily components, namely germ oils, oryzanol and ferula esters.

The compressed and degreased matter may then be dried by heating at elevated temperature for as short a time as possible to prevent any contamination with impurities. Subsequently, the dried matter is extracted with water of an acidic pH of 7 or below, to obtain a composition according to the present invention.

The extraction should preferably be carried out while an aqueous solution of hydrochloric acid of about pH 1 to 3 is added to the dried matter and a temperature of 80 °C or higher is applied thereto.

In order to obtain the present composition from the resulting aqueous extract or phase, it may be dried as such to a solid. Alternatively, ethanol may be added to the aqueous extract at a concentration of, e. g., 40% or higher, which is then concentration or dried after the removal of proteinaceous sediments.

The thus obtained composition according to the present invention contains inositol phosphate compounds as a primary ingredient, and further includes some amounts of saccharides and slight amounts of proteins, fats and inorganic salts.

According to the present inventor's studies, the inositol phosphate compounds have been identified as phosphoric esters of inositol namely mono- to hexa-phosphates.

In order to use the present compositions as internal medicines (for oral administration), they may be formulated into capsules, lemonade preparations, drinkable preparations or the like.

The compositions of the present invention do not cause a lowering of the absorption of inorganic ions essential to human metabolism (which is a defect of phytic acid), and produce an increased action upon the removal of uraroma and body smell, detoxication, the treatment of diabetes and hyperlipemia, the remediation of erythroclasis, dysmnesia and hypohepatia and the inhibition of the proliferation of fat cells.

In particular, the present compositions are most effective in the form of internal application types of uraroma and body smell removers and antidotes for alcohol and drugs acting on central systems.

Although varying depending upon the purposes and preparations, the present compositions may be administered to human beings, generally to adults, at a dose of 1 to 500 mg, calculated as inositol phosphates.

## EXAMPLES

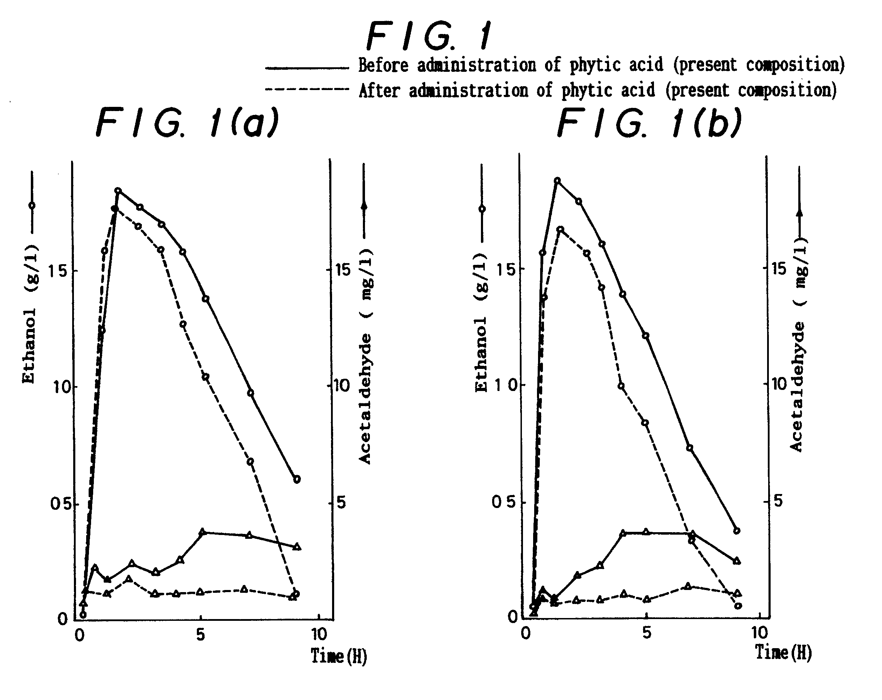

The present invention will now be illustrated specifically by the following examples and experiments. Percentages are be weight. In the drawing Figure 1 illustrates changes-with-time, in Experiment 3, in the concentrations of ethanol and acetaldehyde in the plasma of rabbits to which ethanol was administered with phytic acid and the present preparation, in which:

Figure 1(a) is a graph of the results obtained by the administration of phytic acid alone, and

Figure 1(b) shows the results obtained by the administration of the present preparation.

### EXAMPLE 1

Germ oils, oryzanol and ferula esters were squeezed out of cereal shells and germs (rice bran) by compression to obtain a compressed matter, which was then dried by heating. The thus dried matter was used as the starting material.

Added to 1 kg of the starting material were 1200 cc of water, and a suitable amount of an aqueous solution of hydrochloric acid was added to the resulting aqueous solution to adjust its pH to about 1 to 3. Then, the solution was warmed to 80°C or higher and stirred for one hour. After the removal of solids and insoluble matters by compression, the obtained crude liquid extract was boiled for about 10 minutes

This concentration had to be carried out under such conditions that the pH of the solution was always maintained at 6 or below.

Precipitation was again effected to obtain a liquid extract after the filtering-off of suspended matters.

Added to and mixed with 100 ml of the liquid extract were 300 ml of water and 760 g of dextrin, and the resulting mixture was spray-dried to obtain 785 g of powder.

Contained in 100 ml of the above liquid extract were less than 2 wt % of saccharides, less than 0.2 % of crude proteins and less than 0.1 % of crude fats in addition to 500 mg or more of inositol phosphate compounds.

The instant dry powders are used as such in the form of nutrient enrichers for foodstuffs, taste enrichers for salted vegetables and so on.

### Example 2 - Liquid Refined Product

Added to 100 ml of the liquid extract obtained in similar manner as in Ex 1 was ethanol at a concentration of 40 % or more. After the removal of sediments (proteinous materials), the solution was concentrated under reduced pressure to obtain 20 ml of a concentrated liquid.

### Example 3 - Solid Refined Product

In order to prepare a solid composition, 20 ml of the concentrated liquid obtained in Ex. 2 were diluted with refined water, and 4 g of dextrin were added and absorbed to and kneaded with the solution, which was then dried to obtain 5 g of a solid product.

| Example 4 - Tablets | |
|---|---|
| Composition of Ex 3 (about 10 mg, calculated as inositol phosphate compounds) | 100 mg |
| Corn starch | 19 mg |
| Microcrystalline cellulose | 30 mg |
| Magnesium stearate | 1 mg |

The predetermined amounts of the above ingredients were uniformly mixed together and then compression-molded into tablets, each of 7 mm in diameter and 150 mg in weight.

| Example 5 - Capsules | |
|---|---|
| Composition of Ex 3 (about 20 mg, calculated as inositol phosphate compounds) | 200 mg |
| Lactose | 20 mg |
| Corn starch | 38 mg |
| Magnesium stearate | 2 mg |

The predetermined amounts of the above ingredients were uniformly fixed together and packed in a No. 2 capsule.

| Example 6 - Drinkable Preparation | |
|---|---|
| Concentrated liquid of Ex. 2 (about 200 mg, calculated as inositol phosphate compounds) | 8 ml |
| Honey | 1 mg |
| White sugar | 3 mg |
| Citric acid | suitable amount |
| Sodium citrate | suitable amount |
| Peppermint | suitable amount |
| Refined water | suitable amount |

The predetermined amount of the above ingredients were uniformly mixed together into a colorless, transparent preparation of the internal application type. A 50 ml dose of this liquid preparation contains about 200 mg of inositol phosphate compounds.

Experiment 1 - Removal of Uraroma

To make rats discharge urine, smelling like cat's urine, feed for cats having a high protein content (30 %) was given thereto. That is, sodium phytate (3 mg/day, calculated as phytic acid) and 0.2 ml of the present composition of Ex. 2 (about 5 mg, calculated as inositol phosphate compounds) were intraperitoneally administered to the first and second test groups of rats, respectively, over a period of five days, while no drug was given to a control group of rats, so as to investigate and compare uraroma.

The results are set forth in Table 1, from which it is found that uraroma is removed to a considerable extent in both the first and second test groups; this means that the present composition has a much increased action upon the removal of uraroma.

4

Table 1

| Uraroma | | | | | | |
|---|---|---|---|---|---|---|
| | a | b | c | d | e | f |
| Control Group | + + | + + | + + | + + | + + | + + |
| Test Group 1 | + + | + | ± | ± | ± | + |
| Test Group 2 | + + | ± | - | - | ± | + |

a: before administration,

b: before 3 days,

c: before 4 days,

d: 5 days,

e: 6 days and

f: 9 days.

The results were graded on the scale of + +, +, ± and -.

Experiment 2

For experimentation, pre-fed Afghan long-haired dogs, three in each group, confirmed to be normal by general physical examination, were used. A test group of animals was fed under normal conditions with feed containing the spray-dried powders of Ex. 2 in an amount of 1 g per 1 kg of weight once a day over one week.

By having a total of three inspectors make an inspection of the sebum smells (doggy smells) of the back and maned regions of the animals, smelling tests were carried out, in which it was judged to be significant when, of the three inspectors, two found a significant difference between the test and control groups. As a result, all the inspectors concluded that the sebum smells (doggy smells) were completely or considerably removed from all the animals; this means that the present composition is effective for the removal of body smell.

Experiment 3 - Detoxication of Alcohol

After being fasted for 24 hours, 2 g/kg of alcohol were orally administrated to white rabbits weighing about 3 kg, one for each group, to determine the concentrations of alcohol and aldehyde with time. By way of an intraperitoneal route, 1 ml/kg of the present composition of Ex. 2 (about 25 mg/kgl calculated as inositol phosphate compound) and 20 mg/kg of phytic acid were continuously administered to the same rabbits. After fasting for 24 hours, the animals were tested in similar manner as described above. To this end, 3 mg/ml of sodium citrate were added to blood gathered from the auricular veins to separate plasma, which was then tested with the following kits.

(a) Alcohol In Blood

F Kit for Ethanol, made by BM Yamanouchi Pharmaceutical Co., Ltd.

(b) Acetaldehyde in Blood

F Kit for Acetaldehyde, made by Yamanouchi Pharmaceutical Co., Ltd.

The results are set forth in Figure 1 from which it has been confirmed that the concentrations of alcohol and acetaldehyde in blood are lower after the administration of the present composition and phytic acid than before. It has also been noted that there is a significant difference between the administration of the present composition and the administration of phytic acid alone.

Experiment 4 - Remediation of Medicinal Poisoning

Within 10 minutes after anesthesia had been applied to mice, six in each group, by the intraperitoneal administration of 80 mg/kg of hexabarbital, potassium phytate (5 mg/kg, calculated as phytic acid) was intraperitoneally administered to a test group (1) of mice, while the present composition of Ex. 1 (5 mg/kg, calculated as phytic acid) was similarly administered to a test group (2) of mice, to compare their awakening time. The test group (1) showed a 30 % or more reduction in this time, while the test group (2) exhibited a 35 % or more reduction. From this, it is found that the present composition is slightly more effective than potassium phytate.

## Claims

1. A method of obtaining a composition containing as a primary ingredient inositol phosphate compounds, which comprises degreasing of a cereal the shells and germs by compression, drying the compressed matter by heating, and extracting the dried matter with water of an acidic pH of 7 or less.

2. A method as claimed in Claim 1, wherein said cereal shells and germs are rice bran.

3. A method as claimed in Claim 1 or 2, wherein extraction is carried out while aqueous hydrochloric acid of pH 1 to 3 is added and at a temperature of $80^\circ$ C or higher.

4. A composition containing inositol phosphate compounds as a primary ingredient, when obtained by the method of Claim 1, 2 or 3.

5. A composition as claimed in Claim 4, wherein said inositol phosphate compounds are mono- to hexa-phosphates.

6. A pharmaceutical product prepared from a composition as claimed in Claim 4 or 5.

7. A pharmaceutical product as claimed in Claim 4,5 or 6, for oral administration.

8. Use of a product as claimed in Claim 4,5,6 or 7 for removal of body smell.

9. Use of a composition as claimed in Claim 4,5,6 or 7 as an antidote to the effect of alcohol or drugs on the central nervous system.

# FIG. 1

——— Before administration of phytic acid (present composition)
- - - - - - After administration of phytic acid (present composition)

## FIG. 1(a)

## FIG. 1(b)

EP 0 398 525 A1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | US-A-3270064 (YANOSUKE INABA) <br> * column 1, lines 18 - 28 * <br> * column 1, lines 42 - 59 * <br> * column 2, lines 3 - 19 * | 1-3 | A61K 35/78 <br> A61K 31/66 |
| X | CHEMICAL ABSTRACTS, vol. 105, no. 14, 6 October 1986 <br> Columbus, Ohio, USA <br> OOKAWA,NOBUMASA: "PHYTIN FOR TREATMENT AND PREVENTION OF URINARY CALCULI." <br> page 370; column 1-2; ref. no. 120734 <br> & JP-A-61109730,28-05-1986 <br> * abstract * | 1-3 | |
| X | CHEMICAL ABSTRACTS, vol. 79, no. 3, 23 July 1973 <br> Columbus, Ohio, USA <br> FUJIE SHOSAKU: "PHYTIC ACID" <br> page 483; column 1; ref. no. 19035&JP-A-7307120, 2-03-1973 <br> * abstract * | 1-3 | |
| A | DE-A-667268 (MOKOTOWSKA FABRYCA CHEM.) <br> * the whole document * | 1-3 | TECHNICAL FIELDS SEARCHED (Int. Cl.5 ) <br><br> A61K |
| X,P | EP-A-326963 (SANWA KAGAKU KENKYUSHO) <br> * the whole document * | 4-8 | |
| X,P | EP-A-341810 (SANWA KAGAKU KENKYUSHO) <br> * the whole document * | 4-7, 9 | |

The present search report has been drawn up for all claims

| Place of search | Date of completion of the search | Examiner |
|---|---|---|
| THE HAGUE | 10 SEPTEMBER 1990 | FERNANDEZ Y BRA F. |

EPO FORM 1503 03.82 (P0401)